# EUROPÄISCHE PATENTSCHRIFT

(11) **EP 0 953 358 B1**
(45) Veröffentlichungstag und Bekanntmachung des Hinweises auf die Patenterteilung: **03.01.2007**
(21) Anmeldenummer: 99106026.0
(22) Anmeldetag: 25.03.1999
(51) Int. Cl.: A61K 47/32, A61K 9/00, A61K 9/20, A61K 9/22, C08F 226/02, C08F 226/06, C08F 226/10

(54) **Verwendung von N-Vinyllactam und/oder N-Vinylamin haltigen Copolymeren als Matrix zur Herstellung von festen pharmazeutischen und/oder kosmetischen Darreichungsformen**
Use of coplymers comprising N-Vinyllactam and/or N-Vinylamine as matrix for preparing solid pharmaceutical and/or application forms
Utilisation des copolymères comprenant du N-Vinyllactame et/ou N-Vinylamine comme matrice pour la préparation des produits pharmaceutiques et/ou cosmétiques solides

(30) Priorität: 02.04.1998 DE 19814730
(43) Veröffentlichungstag der Anmeldung: 03.11.1999
(73) Patentinhaber: BASF Aktiengesellschaft, 67056 Ludwigshafen (DE)
(72) Erfinder: Meffert, Helmut, Dr., 68161 Mannheim (DE); Ruchatz, Folker, Dr., 67433 Neustadt (DE)

(56) Entgegenhaltungen:
- EP-A- 0 054 279
- EP-A- 0 409 383
- EP-A- 0 545 209
- EP-A- 0 876 819
- DE-A- 2 514 100
- DE-A- 3 810 343

## Beschreibung

Die vorliegende Erfindung betrifft die Verwendung von N-Vinyllactam oder N-Vinylamin haltigen Copolymeren als Matrix zur Herstellung von festen pharmazeutischen und kosmetischen oralen Darreichungsformen.

Orale Arzneiformen mit einer verzögerten Wirkstofffreigabe (Retardarzneiformen) gewinnen zunehmend an Bedeutung. Mit ihr sind vorteilhafterweise eine verbesserte Patientencompliance durch eine reduzierte Einnahmefrequenz, eine Verringerung von Nebenwirkungen durch Vermeidung von Plasmaspiegelspitzen, gleichmäßigere Blutspiegel des Arzneistoffs sowie die Vermeidung von lokalen Irritationen verbunden.

Neben Coating-Retardformen, d.h. Formulierung von arzneistoffhaltigen Kernen, die mit einem wasserunlöslichen aber semipermeablen bzw. porenhaltigen Film überzogen werden, durch die der Arzneistoff diffundiert, kann die Steuerung und Verlängerung der Freisetzung auch durch eine Einbettung des Arzneistoffes in einer Matrix erreicht werden.

Besonders die Einbettung des Arzneistoffs in eine Matrix bietet die Vorteile einer einfachen und preiswerten Herstellung und einer hohen Arzneimittelsicherheit, da Dose Dumping Effekte (z.B. das Auftreten hoher Plasmakonzentrationen durch falsche Einnahme - beispielsweise das Zerkauen statt Schlucken von gecoateten Tabletten) nicht auftreten können.

Die hierfür in der Regel eingesetzten Hilfsstoffe wie Hydroxypropylcellulose, Hydroxypropylmethylcellulose, Alginsäure bzw. Alginate sowie Xanthan besitzen jedoch anwendungstechnische Nachteile. Diese liegen zum einen in einer pH-Wert- bzw. Ionenstärkeabhängigen Arzneistofffreigabe und zum anderen in einer unbefriedigenden Direkttablettierbarkeit, da aufgrund der geringen Bindemittelwirkung und der schlechten Fließeigenschaften der oben genannten Polymere die resultierenden Preßlinge häufig nur eine geringe Härte aufweisen bzw. inhomogen sind.

Da es sich bei den oben genannten Hilfsstoffen teilweise um Produkte natürlichen Ursprungs bzw. um weiterveredelte Produkte natürlichen Ursprungs handelt, kann es zu Schwankungen in der Chargenkonformität und somit zu einer ungünstigen Beeinflussung der Performance der pharmazeutischen Zubereitung kommen.

In DE-A-25 14 100 werden Copolymere aus Vinylpyrrolidon und langkettigen Alkyl(meth)acrylaten sowie Terpolymere aus Vinylpyrrolidon, Vinylacetat und langkettigen Alkyl(meth)acrylaten zur Verwendung als Emulgatoren für Emulsionen vom Wasser in Öl (W/O)-Typ beschrieben.

DE-A-38 10 343 beschreibt ein Verfahren zur Herstellung von festen pharmazeutischen Retardformen, die als Bindemittel ein N-Vinylpyrrolidon-haltiges wasserlösliches Polymer enthalten, wobei man den pharmazeutischen Wirkstoff, das polymere Bindemittel und gegebenenfalls weitere galenische Hilfsstoffe unterhalb der Glastemperatur des Bindemittels mischt, diese Mischung oberhalb der Glastemperatur des Bindemittels, aber unterhalb der Zersetzungstemperatur des Wirkstoffs zu pharmazeutischen Formen verpreßt und bei einer Temperatur der Form unterhalb der Glastemperatur des Bindemittels entformt.

DE-A-25 28 068 beschreibt vernetzte, wasserunlösliche hydrophile Gele, bestehend u.a. aus N-Vinylpyrrolidon-haltigen Copolymeren mit verschiedenen wasserunlöslichen Monomeren. Als wasserunlösliche Monomere werden Alkylacrylate und Alkylmethacrylate genannt, wobei Alkyl bis zu 18 C-Atome aufweist. Die Polymerisation wird in Masse oder in Lösung durchgeführt, wobei der Wirkstoff während der Polymerisation bereits im Reaktionsmedium vorliegt.

WO 89/06957 beschreibt nicht vernetzte Copolymerisate aus hydrophilen Monomeren wie N-Vinylpyrrolidon, Acrylsäure, Methacrylsäure (und deren niedere Ester und Hydroxyalkylester) und ungesättigten linearen oder verzweigten Estern der Acrylsäure oder Methacrylsäure als hydrophobe Komponente zur Verwendung als Bindemittel für Wirkstoffe, die verzögert freigesetzt werden sollen. Das Molverhältnis der Monomere liegt bei N-Vinylpyrrolidon/Methylmethacrylat von 1 : 1,2 bis 1 : 0,8.

EP-A-0 054 279 sowie EP-A-0 409 383 beschreiben die Verwendung wasserunlöslicher, nicht vernetzter Copolymere aus N-Vinylpyrrolidon und linearen Alkylestern der (Meth)acrylsäure in Zubereitungen zur transdermalen Verabreichung (Salben, Gelen) von Nitroglycerin bzw. Östrogenen.

Der vorliegenden Erfindung lag die Aufgabe zugrunde, Polymere zu finden, die als Matrix zur Herstellung von festen pharmazeutischen und kosmetischen Zubereitungen mit kontrollierter Wirkstofffreigabe geeignet sind.

Diese Aufgabe wurde gelöst durch die Verwendung von Copolymeren, enthaltend
a) 50 bis 99 Gew.-% mindestens eines N-Vinyllactams oder N-Vinylamins, ausgewählt aus der Gruppe, bestehend aus N-Vinylpyrrolidon, N-Vinylpiperidon, N-Vinylcaprolactam, N-Vinylimidazol und methyliertem N-Vinylimidazol oder N-Vinylformamid und
b) 1 bis 50 Gew.-% mindestens eines Monomeren ausgewählt aus der Gruppe der
   b₁) C₁₄-C₃₀-Alkylester von monoethylenisch ungesättigten C₃-C₈-Carbonsäuren;
   b₂) N-C₈-C₃₀-Alkyl-substituierten Amide von monoethylenisch ungesättigten C₃-C₈-Carbonsäuren;
   b₃) N,N-C₈-C₃₀-Dialkyl-substituierten Amide von monoethylenisch ungesättigten C₃-C₈-Carbonsäuren;
   b₄) Vinylester von aliphatischen C₈-C₃₀-Carbonsäuren;
   b₅) C₈-C₃₀-Alkylvinylether
   als Matrix zur Herstellung von festen pharmazeutischen und kosmetischen oralen Darreichungsforrnen.

Als hydrophile Komponenten a) seien folgende polymerisierbare Comonomere genannt:

N-Vinyllactame und N-Vinylamine, insbesondere N-Vinylpyrrolidon, N-Vinylpiperidon, N-Vinylcaprolactam, N-Vinylimidazol, N-Vinyl-2-Methylimidazol, N-Vinyl-4-Methylimidazol sowie N-Vinylformamid.

Bevorzugte hydrophile Komponenten sind N-Vinylpyrrolidon, N-Vinylimidazol und N-Vinylcaprolactam, besonders bevorzugt N-Vinylpyrrolidon.

Der Anteil der hydrophilen Monomerbausteine a) im Copolymerisat liegt im Bereich von 50 bis 99 Gew.-%, bevorzugt 60 bis 99 Gew.-%, besonders bevorzugt im Bereich von 65 bis 98 Gew.-%.

Als hydrophobe Komponenten b) seien folgende polymerisierbare Comonomere genannt:

Ester monoethylenisch ungesättigter C₃-C₈-Carbonsäuren mit einem C₁₄-C₃₀-Alkohol, bevorzugt einem C₁₄-C₂₂-Alkohol, besonders bevorzugt mit einem C₁₄-C₁₈-Alkohol.

Unter monoethylenisch ungesättigten Carbonsäuren mit 3 bis 8 C-Atomen sind beispielsweise Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure zu verstehen.

Aus dieser Gruppe von Carbonsäuren werden bevorzugt Acrylsäure, Methacrylsäure, Maleinsäure oder Mischungen der genannten Carbonsäuren, insbesondere Acrylsäure verwendet.

Besondere Bedeutung kommt hierbei den Acryl- bzw. Methacrylsäureestern mit Fettalkoholen einer Kettenlänge von 14 bis 22 Kohlenstoffatomen zu.

Bevorzugt seien hier genannt: Myristylacrylat, Cetylacrylat, Stearylacrylat, Oleylacrylat, Behenylacrylat, Myristylmethacrylat, Cetylmethacrylat, Stearylmethacrylat, Oleylmethacrylat, Behenylmethacrylat, wobei aus dieser Gruppe besonders die C₁₄-C₁₈-Alkylester der Acrylsäure bevorzugt sind.

Als weitere hydrophobe Comonomere b) können N-C₈-C₃₀-Alkyl- oder N,N-C₈-C₃₀-Dialkyl-substituierte Amide von monoethylenisch ungesättigten C₃-C₈-Carbonsäuren eingesetzt werden, wobei es sich bei den Alkylresten um aliphatische oder cycloaliphatische Alkylreste mit 8 bis 30, bevorzugt 8 bis 22, besonders bevorzugt 12 bis 18 Kohlenstoffatomen handelt.

Die amidierten monoethylenisch ungesättigten Carbonsäuren mit 3 bis 8 C-Atomen können, wie bereits oben genannt, beispielsweise für Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure stehen.

Aus dieser Gruppe von Carbonsäuren werden ebenfalls bevorzugt Acrylsäure, Methacrylsäure, Maleinsäure oder Mischungen der genannten Carbonsäuren verwendet.

Bevorzugte amidierte Comonomere sind beispielsweise N-Octylacrylamid, N-2-Ethylhexylacrylamid, N-Nonylacrylamid, N-Decylacrylamid, N-Laurylacrylamd, N-Myristylacrylamid, N-Cetylacrylamid, N-Stearylacrylamid, N-Oleylacrylamid, N-Behenylacrylamid, N-Octylmethacrylamid, N-2-Ethylhexylmethacrylamid, N-Nonylmethacrylamid, N-Decylmethacrylamid, N-Laurylmethacrylamd, N-Myristylmethacrylamid, N-Cetylmethacrylamid, N-Stearylmethacrylamid, N-Oleylmethacrylamid, N-Behenylmethacrylamid, wobei aus dieser Gruppe besonders die C₁₂-C₁₈-Alkylamide hervorzuheben sind.

Als weitere zusätzliche Komponente b) können Vinylester langkettiger aliphatischer, gesättigter oder ungesättigter C₈-C₃₀-Carbonsäuren, wie z.B. Caprylsäure, Caprinsäure, Laurinsäure, Myristinsäure, Palmitinsäure, Stearinsäure, Arachinsäure, Behensäure, Lignocerinsäure, Cerotinsäure sowie Melissensäure eingesetzt werden. Bevorzugt finden Vinylester der oben genannten C₈-C₁₈-Carbonsäuren Verwendung.

Ferner können C₈-C₃₀-Alkyl-Vinylether, bevorzugt C₈-C₁₈-AlkylVinylether einpolymerisiert werden.

Als bevorzugte C₈-C₁₈-Alkylreste der Vinylether seien unverzweigte Alkylketten wie z.B. n-Octyl, 2-Ethylhexyl, n-Nonyl, n-Decyl, n-Undecyl, n-Dodecyl, n-Tridecyl, n-Tetradecyl, n-Pentadecyl, n-Hexadecyl, n-Heptadecyl sowie n-Octadecyl genannt.

Der Anteil der hydrophoben Monomerbausteine b) im Copolymerisat liegt im Bereich von 1 bis 50 Gew.-%, bevorzugt 1 bis 40 Gew.-%, besonders bevorzugt im Bereich von 2 bis 35 Gew.-%.

Selbstverständlich können auch Mischungen aus zwei oder mehreren Carbonsäureestern, Carbonsäureamiden, Alkylvinylethern oder Vinylestern eingesetzt werden, solange die Summe der Anteile dieser Comonomere nicht 50 Gew-% überschreitet.

Gegebenenfalls kann es sinnvoll sein, neben den bereits genannten Monomerbausteinen a) und b) die im folgenden aufgezählten Comonomere c) für die Polymerisation zu verwenden:

Monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren.

Die monoethylenisch ungesättigten Carbonsäuren können in Form der freien Säure und - soweit vorhanden - der Anhydride oder in partiell oder in vollständig neutralisierter Form bei der Copolymerisation eingesetzt werden. Zur Neutralisation verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetra-ethylenpentamin.

Weitere geeignete Comonomere c) sind beispielsweise die Ester, Amide und Nitrile der oben angegebenen Carbonsäuren, z.B. Acrylsäuremethylester, Acrylsäureethylester, Methacrylsäure-methylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Acrylamid, Methacrylamid, N-Dimethylacrylamid, N-tert.-Butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren sowie die quaternierten Produkte.

Außerdem eignen sich als andere copolymerisierbare Monomere Acrylamidoglycolsäure, Vinylsulfonsäure, Allylsulfonsäure, Methallylsulfonsäure, Styrolsulfonsäure, Acrylsäure-(3-sulfopropyl)ester, Methacrylsäure(3-sulfopropyl)ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure.

Der Anteil der Monomerbausteine c) im Copolymerisat kann im Bereich von 0 bis 30 Gew.-%, bevorzugt 0 bis 20 Gew.-%, besonders bevorzugt im Bereich von 0 bis 10 Gew.-% liegen, wobei sich die Gew.-% Angaben der Komponenten a) bis c) zu 100% addieren.

Bevorzugte Verwendung finden wasserunlösliche Copolymere, enthaltend
a) 60 bis 99 Gew.-% N-Vinylpyrrolidon und
b) 1 bis 40 Gew.-% mindestens eines Monomeren ausgewählt aus der Gruppe der
   b₁) C₁₄-C₁₈-Alkylester von monoethylenisch ungesättigten C₃-C₈-Carbonsäuren;
   b₂) N-C₁₂-C₁₈-Alkyl-substituierten Amide von monoethylenisch ungesättigten C₃-C₈-Carbonsäuren;
   b₃) N,N-C₁₂-C₁₈-Dialkyl-substituierten Amide von monoethylenisch ungesättigten C₃-C₈-Carbonsäuren;
   b₄) Vinylester von aliphatischen C₁₂-C₁₈-Carbonsäuren;
   b₅) C₈-C₁₈-Alkylvinylether.

Die Herstellung der Copolymerisate erfolgt nach bekannten Verfahren, z.B. der Lösungs-, Fällungs-, Emulsions- oder umgekehrte Suspensionspolymerisation unter Verwendung von Verbindungen, die unter den Polymerisationsbedingungen Radikale bilden.

Die Polymerisationstemperaturen liegen üblicherweise in dem Bereich von 30 bis 200, vorzugsweise 40 bis 110°C.

Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Die Copolymeren besitzen K-Werte von mindestens 20, vorzugsweise 25 bis 100, besonders bevorzugt 30 - 80. Die K-Werte werden bestimmt nach H. Fikentscher, Cellulose-Chemie, Band 13, 58 bis 64 und 71 bis 74 (1932) in wäßriger oder alkoholischer Lösung bei 25°C, bei Konzentrationen, die je nach K-Wert-Bereich zwischen 0,1% und 5% liegen.

Das mittlere Molekulargewicht der erfindungsgemäßen Polymere liegt im Bereich von 30.000 bis 10.000.000, bevorzugt 35.000 bis 2.000.000, besonders bevorzugt von 40.000 bis 1.500.000.

Die erhaltenen Polymer-Dispersionen oder Lösungen können durch verschiedene Trocknungsverfahren wie z.B. Sprühtrocknung, Fluidized Spray Drying, Walzentrocknung oder Gefriertrocknung in Pulverform überführt werden, aus der sich durch Redispergieren in Wasser erneut eine wäßrige Dispersion herstellen läßt.

Die Polymere eignen sich erfindungsgemäß als Matrix zur Herstellung von festen kosmetischen und pharmazeutischen oralen Darreichungsformen bei denen der oder die Wirkstoffe verzögert freigegeben werden sollen.

Je nach Menge und Zusammensetzung und der daraus resultierenden Quellungseigenschaften der erfindungsgemäß verwendeten Polymere läßt sich dabei die Freisetzungsgeschwindigkeit der Wirkstoffe gezielt verändern.

Gegenstand der Erfindung sind daher auch pharmazeutische und kosmetische Zubereitungen, enthaltend als Matrix mindestens ein Copolymer aus
a) 50 bis 99 Gew.-% mindestens eines N-Vinyllactams oder N-Vinylamins, ausgewählt aus der Gruppe, bestehend aus N-Vinylpyrrolidon, N-Vinylpiperidon, N-Vinylcaprolactam, N-Vinylimidazol und methyliertem N-Vinylimidazol oder N-Vinylformamid und
b) 1 bis 50 Gew.-% mindestens eines Monomeren ausgewählt aus der Gruppe der
   b₁) C₁₄-C₃₀-Alkylester von monoethylenisch ungesättigten C₃-C₈-Carbonsäuren;
   b₂) N-C₈-C₃₀-Alkyl-substituierten Amide von monoethylenisch ungesättigten C₃-C₈-Carbonsäuren;
   b₃) N,N-C₈-C₃₀-Dialkyl-substituierten Amide von monoethylenisch ungesättigten C₃-C₈-Carbonsäuren;
   b₄) Vinylester von aliphatischen C₈-C₃₀-Carbonsäure;
   b₅) C₈-C₃₀-Alkylvinylether.

Bevorzugt sind solche pharmazeutische und kosmetische Zubereitungen, enthaltend als Matrix mindestens ein Copolymer aus
a) 60 bis 99 Gew.-% N-Vinylpyrrolidon und
b) 1 bis 40 Gew.-% mindestens eines Monomeren ausgewählt aus der Gruppe der
   b₁) C₁₄-C₁₈-Alkylester von monoethylenisch ungesättigten C₃-C₈-Carbonsäuren;
   b₂) N-C₁₂-C₁₈-Alkyl-substituierten Amide von monoethylenisch ungesättigten C₃-C₈-Carbonsäuren;
   b₃) N,N-C₁₂-C₁₈-Dialkyl-substituierten Amide von monoethylenisch ungesättigten C₃-C₈-Carbonsäuren;
   b₄) Vinylester von aliphatischen C₁₂-C₁₈-Carbonsäuren;
   b₅) C₈-C₁₈-Alkylvinylether.

Für die allgemeine und bevorzugte Definition der einzelnen Monomerbausteine a) und b) sowie für die Zusammensetzung und das Molekulargewicht der erfindungsgemäßen Copolymere ist die bereits eingangs erfolgte Beschreibung heranzuziehen.

Die erfindungsgemäßen festen pharmazeutischen oder kosmetischen Zubereitungen sind dadurch gekennzeichnet, daß sie das als Matrix dienende Copolymer in einer Konzentration von 0,5 bis 90 Gew.-%, bevorzugt 20 bis 80 Gew.-%, besonders bevorzugt von 30 bis 80 Gew.-% enthalten.

Die Zubereitungen lassen sich nach an sich bekannten Methoden u.a. durch Direkttablettierung, Trockengranulation oder Feuchtgranulation sowie Feuchtextrusion der erfindungsgemäßen Copolymere mit dem pharmazeutischen oder kosmetischen Wirkstoff herstellen. Eine bevorzugtes Verfahren zur Herstellung der festen pharmazeutischen oder kosmetischen Zubereitungen ist dabei die Direkttablettierung.

Je nach Zusammensetzung der erfindungsgemäßen Copolymere lassen sich pharmazeutische oder kosmetische Zubereitungen herstellen, bei denen die Freisetzung der Wirkstoffe innerhalb einer Zeit von 0,25 bis 24 Stunden, bevorzugt 0,5 bis 20 Stunden, besonders bevorzugt innerhalb von 2 bis 16 Stunden erfolgt.

Als pharmazeutische Wirkstoffe seien hier Arzneimittel beispielsweise aus der Gruppe der Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Bei der Formulierung der Zubereitungen können selbstverständlich auch weitere, für die Herstellung von festen oralen Darreichungsformen übliche Hilfsstoffe zugesetzt werden.

Dies können u.a. sein:
Füllstoffe und Bindemittel wie z.B. Lactose, Calciumphosphate, Cellulose und Cellulosederivate, Stärke und Stärkederivate, Polyvinylpyrrolidon, Polyvinylalkohol, partiell verseiftes Polyvinylacetat, Zuckeralkohole, Zucker, Fette, Wachse; Sprengmittel wie z.B. Kollidon^{®} CL (Fa. BASF), Na-Carboxymethylstärke, Na-Carboxymethylcellulose;
Gleit- und Schmiermittel wie z.B. Mg-stearat, Ca-behenat, Stearinsäure, PEG;
Fließregulierungsmittel wie z.B. hochdisperses Siliciumdioxid;
Filmbildner wie z.B. Polyacrylate und Polymethacrylate (Eudragittypen), Copolymere auf Basis von Acrylatderivaten, Hydroxypropylmethylcellulose, Hydroxypropylcellulose, Celluloseacetat, Celluloseacetatphthalat und andere magensaftresistente Überzugsmaterialien; Feuchthaltemittel wie z.B. Glycerin, Propylenglykol, Sorbitol, Mannitol, Polyethylenglykole sowie
Weichmacher, Farbstoffe, Tenside, Salze, Dispergierhilfsmittel.

In den folgenden Beispielen wird die Verwendung der erfindungsgemäßen Copolymere näher erläutert.

### Beispiel 1

Jeweils 160 mg der in der Tabelle 1 aufgeführten Polymere wurden mit 160 mg Propanolol-HCl, 3,4 mg hochdispersem Siliciumdioxid und 1,6 mg Magnesiumstearat gemischt und anschließend mit einem Preßdruck von 18 KN mit 10 mm Stempeln facettiert und direkttabllettiert.

Die Tabletten wurden in einer Freisetzungsapparatur (Paddle-Apparatus USP XXIII) bei 37 °C und einer Umdrehungsgeschwindigkeit des Rührers von 50 rpm auf ihre Freisetzungseigenschaften geprüft. Der Wirkstoff wurde in 900 ml Phosphatpuffer pH 7,4 (USP XXIII) freigesetzt.

**Tabelle 1**

| Zusammensetzung | Gew.-% | K-Wert*) | Freisetzung nach x min [%] | | | |
|---|---|---|---|---|---|---|
| | | | 60 | 240 | 480 | 960 |
| Vinylpyrrolidon/ Stearylacrylat | 95/5 | 52,4 | 30 | 67 | 92 | --- |
| Vinylpyrrolidon/ Stearylacrylat | 95/5 | 41,8 | 55 | 90 | 90 | --- |
| Vinylpyrrolidon/ Stearylacrylat | 90/10 | 40,7 | 40 | 90 | 95 | --- |
| Vinylpyrrolidon/ Stearylacrylat | 80/20 | 42,7 | 18 | 45 | 80 | 92 |
| Vinylpyrrolidon/ Stearylacrylat | 80/20 | 35,1 | 16 | 47 | 81 | 100 |
| Vinylpyrrolidon/ Stearylacrylat | 90/10 | 35,0 | 40 | 95 | 97 | --- |
| Vinylpyrrolidon/ Stearylacrylat | 80/20 | 34,3 | 30 | 85 | 95 | --- |
| Vinylpyrrolidon/ Stearylmethacrylat | 80/20 | 55,3 | 17,1 | 47,8 | 89,2 | 96,5 |
| Vinylpyrrolidon/ Stearylmethacrylat | 70/30 | 46,1 | 12,4 | 38,7 | 57,6 | 80,9 |
| Vinylformamid/ Stearylacrylat | 70/30 | | 22,4 | 38,1 | 53,9 | 71,6 |

| | | | | | | |
|---|---|---|---|---|---|---|
| *) Die K-Werte wurden in Ethanol bei einer Polymerkonzentration von 1 Gew.-% bestimmt. | | | | | | |

## Patentansprüche

1. Verwendung von Copolymeren, enthaltend
a) 50 bis 99 Gew.-% mindestens eines N-Vinyllactams oder N-Vinylamins, ausgewählt aus der Gruppe, bestehend aus N-Vinylpyrrolidon, N-Vinylpiperidon, N-Vinylcaprolactam, N-Vinylimidazol und methyliertem N-Vinylimidazol oder N-Vinylformamid und
b) 1 bis 50 Gew.-% mindestens eines Monomeren ausgewählt aus der Gruppe der
b₁) C₁₄-C₃₀-Alkylester von monoethylenisch ungesättigten C₃-C₈-Carbonsäuren;
b₂) N-C₈-C₃₀-Alkyl-substituierten Amide von monoethylenisch ungesättigten C₃-C₈-Carbonsäuren;
b₃) N,N-C₈-C₃₀-Dialkyl-substituierten Amide von monoethylenisch ungesättigten C₃-C₈-Carbonsäuren;
b₄) Vinylester von aliphatischen C₈-C₃₀-Carbonsäuren;
b₅) C₈-C₃₀-Alkylvinylether
als Matrix zur Herstellung von festen pharmazeutischen und kosmetischen oralen Darreichungsformen.

2. Verwendung von Copolymeren nach Anspruch 1, enthaltend
a) 60 bis 99 Gew.-% N-Vinylpyrrolidon und
b) 1 bis 40 Gew.-% mindestens eines Monomeren ausgewählt aus der Gruppe der
b₁) C₁₄-C₂₂-Alkylester von monoethylenisch ungesättigten C₃-C₈-Carbonsäuren;
b₂) N-C₈-C₂₂-Alkyl-substituierten Amide von monoethylenisch ungesättigten C₃-C₈-Carbonsäuren;
b₃) N,N-C₈-C₂₂-Dialkyl-substituierten Amide von monoethylenisch ungesättigten C₃-C₈-Carbonsäuren;
b₄) Vinylester von aliphatischen C₈-C₂₂-Carbonsäuren;
b₅) C₈-C₁₈-Alkylvinylether.

3. Verwendung nach Anspruch 1, wobei die Copolymeren 0 bis 30 Gew.-% weiterer radikalisch copolymerisierbarer Monomere c) enthalten.

4. Verwendung von Copolymeren nach den Ansprüchen 1 bis 3, **dadurch gekennzeichnet, daß** sie durch Direkttablettierung, Trockengranulation oder Feuchtgranulation sowie durch Feuchtextrusion mit dem pharmazeutischen oder kosmetischen Wirkstoff verarbeitet werden.

5. Festepharmazeutische und kosmetische orale Zubereitungen, enthaltend als Matrix mindestens ein Copolymer aus
a) 50 bis 99 Gew.-% mindestens eines N-Vinyllactams oder N-Vinylamins, ausgewählt aus der Gruppe, bestehend aus N-Vinylpyrrolidon, N-Vinylpiperidon, N-Vinylcaprolactam, N-Vinylimidazol und methyliertem N-Vinylimidazol oder N-Vinylformamid und
b) 1 bis 50 Gew.-% mindestens eines Monomeren ausgewählt aus der Gruppe der
b₁) C₁₄-C₃₀-Alkylester von monoethylenisch ungesättigten C₃-C₈-Carbonsäuren;
b₂) N-C₈-C₃₀-Alkyl-substituierten Amide von monoethylenisch ungesättigten C₃-C₈-Carbonsäuren;
b₃) N,N-C₈-C₃₀-Dialkyl-substituierten Amide von monoethylenisch ungesättigten C₃-C₈-Carbonsäuren;
b₄) Vinylester von aliphatischen C₈-C₃₀-Carbonsäuren;
b₅) C₈-C₃₀-Alkylvinylether.

6. Pharmazeutische und kosmetische Zubereitungen nach Anspruch 5, enthaltend als Matrix mindestens ein Copolymer aus
a) 60 bis 99 Gew.-% N-Vinylpyrrolidon und
b) 1 bis 40 Gew.-% mindestens eines Monomeren ausgewählt aus der Gruppe der
b₁) C₁₄-C₂₂-Alkylester von monoethylenisch ungesättigten C₃-C₈-Carbonsäuren;
b₂) N-C₈-C₂₂-Alkyl-substituierten Amide von monoethylenisch ungesättigten C₃-C₈-Carbonsäuren;
b₃) N,N-C₈-C₂₂-Dialkyl-substituierten Amide von monoethylenisch ungesättigten C₃-C₈-Carbonsäuren;
b₄) Vinylester von aliphatischen C₈-C₂₂-Carbonsäuren;
b₅) C₈-C₁₈-Alkylvinylether.

7. Feste pharmazeutische oder kosmetische Zubereitungen nach den Ansprüchen 5 und 6, enthaltend das als Matrix dienende Copolymer in einer Konzentration von 0,5 bis 90 Gew.-%.

8. Feste pharmazeutische oder kosmetische Zubereitungen nach den Ansprüchen 5 bis 7, aus denen die Wirkstoffe innerhalb einer Zeit von 0,25 bis 24 Stunden freigesetzt werden.

## Claims

1. The use of copolymers comprising
a) 50 to 99% by weight of at least one N-vinyllactam or N-vinylamine selected from the group consisting of N-vinylpyrrolidone, N-vinylpiperidone, N-vinylcaprolactam, N-vinylimidazole and methylated N-vinylimidazole or N-vinylformamide and
b) 1 to 50% by weight of at least one monomer selected from the group of
b₁) C₁₄-C₃₀-alkyl esters of monoethylenically unsaturated C₃-C₈-carboxylic acids;
b₂) N-C₈-C₃₀-alkyl-substituted amides of monoethylenically unsaturated C₃-C₈-carboxylic acids;
b₃) N,N-C₈-C₃₀-dialkyl-substituted amides of monoethylenically unsaturated C₃-C₈-carboxylic acids;
b₄) vinyl esters of aliphatic C₈-C₃₀-carboxylic acids;
b₅) C₈-C₃₀-alkyl vinyl ethers
as matrix for producing solid pharmaceutical and cosmetic oral presentations.

2. The use of copolymers according to claim 1, comprising
a) 60-99% by weight of N-vinylpyrrolidone and
b) 1-40% by weight of at least one monomer selected from the group of
b₁) C₁₄-C₂₂-alkyl esters of monoethylenically unsaturated C₃-C₈-carboxylic acids;
b₂) N-C₈-C₂₂-alkyl-substituted amides of monoethylenically unsaturated C₃-C₈-carboxylic acids;
b₃) N,N-C₈-C₂₂-dialkyl-substituted amides of monoethylenically unsaturated C₃-C₈-carboxylic acids;
b₄) vinyl esters of aliphatic C₈-C₂₂-carboxylic acids;
b₅) C₈-C₁₈-alkyl vinyl ethers.

3. The use according to claim 1, wherein the copolymers comprise 0-30% by weight of other monomers c) which can undergo free-radical copolymerization.

4. The use of copolymers according to any of claims 1 to 3, wherein they are processed by direct tabletting, dry granulation or wet granulation, and by wet extrusion with the pharmaceutical or cosmetic active ingredient.

5. A solid pharmaceutical or cosmetic oral preparation comprising as matrix at least one copolymer of
a) 50 to 99% by weight of at least one N-vinyllactam or N-vinylamine selected from the group consisting of N-vinylpyrrolidone, N-vinylpiperidone, N-vinylcaprolactam, N-vinylimidazole and methylated N-vinylimidazole or N-vinylformamide and
b) 1 to 50% by weight of at least one monomer selected from the group of
b₁) C₁₄-C₃₀-alkyl esters of monoethylenically unsaturated C₃-C₈-carboxylic acids;
b₂) N-C₈-C₃₀-alkyl-substituted amides of monoethylenically unsaturated C₃-C₈-carboxylic acids;
b₃) N,N-C₈-C₃₀-dialkyl-substituted amides of monoethylenically unsaturated C₃-C₈-carboxylic acids;
b₄) vinyl esters of aliphatic C₈-C₃₀-carboxylic acids;
b₅) C₈-C₃₀-alkyl vinyl ethers.

6. The pharmaceutical or cosmetic preparation according to claim 5, comprising as matrix at least one copolymer of
a) 60-99% by weight of N-vinylpyrrolidone and
b) 1-40% by weight of at least one monomer selected from the group of
b₁) C₁₄-C₂₂-alkyl esters of monoethylenically unsaturated C₃-C₈-carboxylic acids;
b₂) N-C₈-C₂₂-alkyl-substituted amides of monoethylenically unsaturated C₃-C₈-carboxylic acids;
b₃) N,N-C₈-C₂₂-dialkyl-substituted amides of monoethylenically unsaturated C₃-C₈-carboxylic acids;
b₄) vinyl esters of aliphatic C₈-C₂₂-carboxylic acids;
b₅) C₈-C₁₈-alkyl vinyl ethers.

7. The solid pharmaceutical or cosmetic preparation according to claims 5 and 6, comprising the copolymer serving as matrix in a concentration of from 0.5 to 90% by weight.

8. The solid pharmaceutical or cosmetic preparation according to any of claims 5 to 7, from which the active ingredients are released within a period of from 0.25 to 24 hours.

## Revendications

1. Utilisation de copolymères contenant
a) 50 à 99 % en poids d'au moins un N-vinyllactame ou N-vinylamine choisi parmi le groupe constitué de N-vinylpyrrolidone, de N-vinylpipéridone, de N-vinylcaprolactame, de N-vinylimidazole et de N-vinylimidazole méthylé ou de N-vinylformamide, et
b) 1 à 50 % en poids d'au moins un monomère choisi parmi le groupe
b₁) des esters alkyliques en C₁₄-C₃₀ d'acides carboxyliques en C₃-C₈ monoéthyléniquement insaturés,
b₂) des amides à substitution N-C₈-C₃₀-alkylique d'acides carboxyliques en C₃-C₈ monoéthyléniquement insaturés,
b₃) des amides à substitution N,N-C₈-C₃₀-dialkylique d'acides carboxyliques en C₃-C₈ monoéthyléniquement insaturés,
b₄) des esters vinyliques d'acides carboxyliques en C₈-C₃₀ aliphatiques,
b₅) d'éthers alkylvinyliques en C₈-C₃₀,
comme matrice pour la préparation de formes d'administration orale pharmaceutiques et cosmétiques, solides.

2. Utilisation de copolymères suivant la revendication 1, contenant
a) 60 à 99 % en poids de N-vinylpyrrolidone, et
b) 1 à 40 % en poids d'au moins un monomère choisi parmi le groupe
b₁) des esters alkyliques en C₁₄-C₂₂ d'acides carboxyliques en C₃-C₈ monoéthyléniquement insaturés,
b₂) des amides à substitution N-C₈-C₂₂-alkylique d'acides carboxyliques en C₃-C₈ monoéthyléniquement insaturés,
b₃) des amides à substitution N,N-C₈-C₂₂-dialkylique d'acides carboxyliques en C₃-C₈ monoéthyléniquement insaturés,
b₄) des esters vinyliques d'acides carboxyliques en C₈-C₂₂ aliphatiques,
b₅) des éthers alkylvinyliques en C₈-C₁₈.

3. Utilisation suivant la revendication 1, dans laquelle les copolymères contiennent 0 à 30 % en poids d'autres monomères copolymérisables par voie radicalaire c).

4. Utilisation de copolymères suivant les revendications 1 à 3, **caractérisée en ce qu'**ils sont traités par formation directe de comprimés, granulation à l'état sec ou humide ainsi que par extrusion à l'état humide avec la substance active pharmaceutique ou cosmétique.

5. Compositions orales pharmaceutiques et cosmétiques, solides, contenant comme matrice au moins un copolymère à base
a) de 50 à 99 % en poids d'au moins un N-vinyllactame ou N-vinylamine choisi parmi le groupe constitué de N-vinylpyrrolidone, de N-vinylpipéridone, de N-vinylcaprolactame, de N-vinylimidazole et de N-vinylimidazole méthylé ou de N-vinylformamide, et
b) de 1 à 50 % en poids d'au moins un monomère choisi parmi le groupe
b₁) des esters alkyliques en C₁₄-C₃₀ d'acides carboxyliques en C₃-C₈ monoéthyléniquement insaturés,
b₂) des amides à substitution N-C₈-C₃₀-alkylique d'acides carboxyliques en C₃-C₈ monoéthyléniquement insaturés,
b₃) d'amides à substitution N, N-C₈-C₃₀-dialkylique d'acides carboxyliques en C₃-C₈ monoéthyléniquement insaturés,
b₄) des esters vinyliques d'acides carboxyliques en C₈-C₃₀ aliphatiques,
b₅) des éthers alkylvinyliques en C₈-C₃₀.

6. Compositions pharmaceutiques et cosmétiques suivant la revendication 5, contenant, comme matrice, au moins un copolymère à base
a) de 60 à 99 % en poids de N-vinylpyrrolidone, et
b) de 1 à 40 % en poids d'au moins un monomère choisi parmi le groupe
b₁) des esters alkyliques en C₁₄-C₂₂ d'acides carboxyliques en C₃-C₈ monoéthyléniquement insaturés,
b₂) des amides à substitution N-C₈-C₂₂-alkylique d'acides carboxyliques en C₃-C₈ monoéthyléniquement insaturés,
b₃) des amides à substitution N,N-C₈-C₂₂-dialkylique d'acides carboxyliques en C₃-C₈ monoéthyléniquement insaturés,
b₄) des esters vinyliques d'acides carboxyliques en C₈-C₂₂ aliphatiques,
b₅) des éthers alkylvinyliques en C₈-C₁₈.

7. Compositions pharmaceutiques ou cosmétiques solides suivant les revendications 5 et 6, contenant le copolymère servant de matrice en une concentration de 0,5 à 90 % en poids.

8. Compositions pharmaceutiques ou cosmétiques solides suivant les revendications 5 à 7, à partir desquelles les substances actives sont libérées en un espace de temps de 0,25 à 24 heures.
